(19) Europäisches Patentamt
European Patent Office
Office européen des brevets

(11) Publication number: **0 158 694**
**B1**

(12) # EUROPEAN PATENT SPECIFICATION

(45) Date of publication of patent specification: **22.02.89**

(51) Int. Cl.[4]: **C 07 C 51/377**, C 07 C 57/04, C 07 C 67/317, C 07 C 69/54

(21) Application number: **84104418.3**

(22) Date of filing: **18.04.84**

(54) **Oxydehydrogenation process.**

(43) Date of publication of application:
**23.10.85 Bulletin 85/43**

(45) Publication of the grant of the patent:
**22.02.89 Bulletin 89/08**

(84) Designated Contracting States:
**AT BE CH DE FR GB IT LI LU NL SE**

(56) References cited:
**EP-A-0 088 615**
**GB-A- 852 664**
**US-A-3 948 959**
**US-A-4 232 174**
**US-A-4 338 463**
**US-A-4 410 727**

(73) Proprietor: **Ashland Oil, Inc.**
**1401 Winchester Avenue**
**Ashland Kentucky 41101 (US)**

(72) Inventor: **Chelliah, Daniel**
**1460 Slade Avenue**
**Columbus, OH 43220 (US)**

(74) Representative: **Kraus, Walter, Dr. et al**
**Patentanwälte Kraus, Weisert & Partner**
**Thomas-Wimmer-Ring 15**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

Background of the invention

Field of the invention

This invention relates to an improved process for the conversion of isobutyric acid and its lower alkyl esters correspondingly to methacrylic acid and lower alkyl esters thereof.

Description of the prior art

There exists considerable prior art relating to the oxydehydrogenation of the lower saturated aliphatic monocarboxylic acids to produce the corresponding α,β-olefinically unsaturated acids. Early work in this area involved thermal, vapor phase oxydehydrogenation of the saturated aliphatic carboxylic acid in the presence of oxygen and iodine. This approach has not been particularly successful from a commercial standpoint. This is understandably so inasmuch as iodine is costly, exhibits extreme corrosivity properties and poses considerable problems in realizing complete recovery of the comparatively large amounts thereof required in the process. The heterogeneous catalytic method for oxydehydrogenation according to the prior art appears to be the more attractive route to the commercial production of olefinically unsaturated monocarboxylic acids. The prior art heterogeneous oxydehydrogenation catalysts useful for this purpose include some heteropoly acids, such as phosphomolybdic acid, optionally with tungsten and/or vanadium. Another type of catalyst included in the prior art is iron phosphate.

Iron phosphate subjected to calcination exists in several crystalline phases or species. It is not known at this time which species is or are catalytically active. There is evidence that the presence of certain extrinsic metal components in the catalyst preparation serves to facilitate the formation of the active catalyst. For instance, U.S. Patent No. 3,948,959 discloses that an alkali or alkaline earth metal can be the extrinsic metal component for this purpose.

U.S. Patent 4,410,727 is directed to a process for the catalytic conversion of isobutyric acid or a lower alkyl ester thereof to the corresponding α,β-olefinically unsaturated derivative by oxydehydrogenation wherein a catalyst is contacted with a gaseous stream containing said acid or ester and molecular oxygen at a temperature between about 300 and 500°C, and wherein a catalyst of the formula

$$Fe^{II}Fe_2^{III}(PO_4)_2(OH)_2$$

is used which exists in the form of a Barbosalite and/or Lipscombite. This reaction can be carried out in the presence of acetone.

The catalyst used gives a good percent selectivity of isobutyric acid to methacrylic acid. This selectivity is in the range of about 77 to about 82.5%. However, the conversion of the isobutyric acid is rather low and insufficient.

Applicant has now found that, if one uses the special catalysts, the conversion of the isobutyric acid is surprisingly much higher and is in the range of about 70%.

The improvement achieved by using the special type catalysts is unobvious for the man skilled in the art.

Summary of the invention

In accordance with this invention a process is provided for the catalytic conversion of isobutyric acid or ester to methacrylic acid or ester by oxydehydrogenation wherein an oxydehydrogenation catalyst of the iron phosphate type selected from the group consisting of

a. a tellurium containing iron phosphate type catalyst,

b. an iron, cesium phosphate

is contacted with a feed stream containing said acid or ester and molecular oxygen at a temperature between 300 and 500°C, which is characterised in that acetone is included in the feed stream.

Description of the preferred embodiment

Any of several known oxydehydrogenation catalysts can be used in the process of this invention. Typical catalysts are disclosed in U.S. Patent No. 3,948,959 which describes alkali or alkaline earth metal containing iron phosphate catalyst. Such catalysts are conveniently prepared by methods which have been described in the prior art. Oxydehydrogenation catalysts useful in this invention usually are calcined in the presence of air at a temperature in the range of from 400 to 1000°C. Applicable periods of calcination range from 2 to 30 hours or even greater.

The use of a support or carrier for the catalyst is included in this invention. Commonly used support materials include silica, alumina, magnesia, titania, quartz, carbon, silicon carbide, and the like.

The process of this invention can be carried out using the catalyst in the form of a fluidized bed reactor, a stirred tank reactor, or in the fixed bed or packed bed reactor or any combination of these types of reactors. Because of the convenience associated with the use of a fixed bed reactor in a small scale operation, such a reactor will be exemplified herein. In the preferred mode of operation the feed to the reactor comprises a pre-heated gaseous mixture of the isobutyric acid, molecular oxygen, steam, inert diluent gas, and acetone. A pre-heat temperature in the range of about 300 to 350°C is customarily used. The oxydehydrogenation reaction can be carried out in the range of from 300 to 500°C.

The mole ratio of molecular oxygen to the isobutyric acid or ester is from 0.5 to 1.5 and more preferably from 0.7 to 0.75. Although steam is not necessary, its presence is desirable in the feed because it is believed to act beneficially as a heat sink and in minimizing combustion of the isobutyric acid to undesirable waste products. The mole ratio of water to the carboxylic acid in the feed should be from about 2 to 20—the optimum is from 8 to 15.

The inclusion of acetone in the feed in the process of this invention causes enhanced selectivity in production of methacrylic acid from isobutyric acid. The reason for this unexpected phenomenon is not known at this time. The use of acetone in the feed also improves catalyst life. The mole ratio of acetone to isobutyric acid or its lower alkyl ester in the feed should be in the range of from about 0.1 to about 20. More preferably the ratio should be from 0.1 to 5.0, most preferably the ratio should be from 0.18 to 3.0 in the process of this invention.

Another important parameter is the concentration of the isobutyric acid or ester thereof in the feed. The isobutyric acid or ester should be present in the feed in from about 0.1 to 20 mole percent. From the standpoint of achieving a reasonable through-put combined with an acceptable yield, the concentration of the acid or ester in the feed from about 3—6 mole percent concentration is controlled to a large degree by the amount of inert gas present. The preferred inert gas or diluent is nitrogen although other inert gases such as carbon dioxide, helium, argon, and the like are suitable. Air is a very convenient source of oxygen plus inert diluent.

Another important parameter is contact time in the process of this invention. Contact or reaction time is defined for the purpose of this invention as the catalyst volume divided by the volume of gas feed per second at the reaction temperature. The catalyst volume is the bulk volume occupied by the catalyst in the reactor. The term catalyst in this sense not only includes the modified iron phosphate itself for instance, but also the support if present. Accordingly, reaction times can range from 0.05 to 3.0 seconds and more generally in the order of from 0.1 to 1.0 second. The reaction is preferably carried out at or near atmospheric pressure although the use of higher pressures up to 10 atmospheres or even higher is contemplated to be within the scope of this invention.

The process of this invention is further illustrated in the following specific examples.

Example I

A. A mixture of isobutyric acid, water, acetone, oxygen and nitrogen in the mole ratio 1:15.6:0.3:0.5:1.8, respectively, was passed over a calcined catalyst having the empirical formula

$$FeCs_{.15}P_{1.25}O_4/SiO_2$$

3.5% in a fixed bed reactor which was a 1" diameter stainless steel tube 13" in length. The 12—20 mesh catalyst was packed in the tube with inert quartz chips in the weight ratio of 1:2, respectively. The total volume of the bed of catalyst plus quartz was 45 cc. The contact time was 0.5 seconds and the reaction temperature was 410°C. In a continuous reaction, high conversions of isobutyric acid with high selectivity to methacrylic acid were achieved. At the end of 20 hours on stream the conversion of isobutyric acid was 87% with a selectivity of 75% to methacrylic acid and at the end of 431 hours on stream the conversion of isobutyric acid was 87% and selectivity to methacrylic acid was 77.3%.

B. A repeat of A above without the inclusion of acetone gave an 83.5% conversion of isobutyric acid and a selectivity of 71% to methacrylic acid after 29 hours on stream and a conversion of 80.2% of isobutyric acid with a selectivity to methacrylic acid of 68.3% after 125 hours on stream.

The conversion of isobutyric acid represents the mole ratio of isobutyric acid transformed to all other products including methacrylic acid and selectivity to methacrylic acid represents the mole ratio of methacrylic acid formed to that of the isobutyric acid converted.

Example II

A. The procedure of Example I was repeated using a calcined catalyst having the empirical formula

$$Fe_{1.0}Te_{.05}P_{1.3}O_x/SiO_2$$

20% wherein x is a number which will satisfy the unshared positive valences of the other elements present in the formula. Several samples of the reactor effluent were analyzed after the reaction had been on stream for at least 8 hours with the following results:

| Isobutyric acid | % Selectivity to methacrylic acid |
|---|---|
| 71.7 | 78.0 |
| 76.0 | 71.0 |
| 73.0 | 71.0 |

B. A repeat of A of this example with the exclusion of acetone in the feed produced the following results:

| % Conversion of isobutyric acid | % Selectivity to methacrylic acid |
|---|---|
| 73.7 | 54.6 |
| 76.6 | 57.9 |

It was observed in the foregoing examples that essentially all of the acetone used in the feed (99%) could be accounted for in the effluent which indicates that acetone does not act as a reactant.

## Claims

1. A process for the catalytic conversion of isobutyric acid or ester to methacrylic acid or ester by oxydehydrogenation wherein an oxydehydrogenation catalyst of the iron phosphate type selected from the group consisting of

a. a tellurium containing iron phosphate type catalyst,

b. an iron, cesium phosphate
is contacted with a feed stream containing said acid or ester and molecular oxygen at a temperature between 300 and 500°C, characterized in that acetone is included in the feed stream.

2. The process of Claim 1 wherein isobutyric acid is converted to methacrylic acid.

3. The process of Claim 2 wherein the mole ratio of acetone to isobutyric acid is from 0.1 to 5.0.

**Patentansprüche**

1. Verfahren für die katalytische Umwandlung von Isobuttersäure oder ihrem Ester in Methacrylsäure oder ihren Ester durch Oxydehydrierung, wobei ein Oxydehydrierungs-Katalysator des Eisenphosphat-Typs, ausgewählt aus der Gruppe

a. einem Tellur enthaltenden Katalysator des Eisenphosphat-Typs,

b. einem Eisencäsiumphosphat,
mit einem Beschickungsstrom, der die Säure oder den Ester und molekularen Sauerstoff enthält, bei einer Temperatur zwischen 300 und 500°C behandelt wird, dadurch gekennzeichnet, daß Aceton zu dem Beschickungsstrom zugegeben wird.

2. Verfahren nach Anspruch 1, dadurch gekennzeichnet, daß Isobuttersäure in Methacrylsäure umgewandelt wird.

3. Verfahren nach Anspruch 2, dadurch gekennzeichnet, daß das Molverhältnis von Aceton zu Isobuttersäure von 0,1 bis 5,0 beträgt.

**Revendications**

1. Un procédé pour la conversion catalytique de l'acide ou d'un ester isobutyric en acide ou ester méthacrylique par oxydéshydrogénation, dans lequel on met en contact un catalyseur d'oxydéshydrogénation du type phosphate de fer choisi parmi

a) un catalyseur du type phosphate de fer contenant du tellure et

b) un phosphate de fer, césium
avec un courant d'alimentation contenant ledit acide ou ester et de l'oxygène moléculaire à une température comprise entre 300 et 500°C, caractérisé en ce que le courant d'alimentation contient de l'acétone.

2. Le procédé selon la revendication 1, dans lequel l'acide isobutyrique ester converti en acide méthacrylique.

3. Le procédé selon la revendication 2, dans lequel le rapport molaire de l'acétone à l'acide isobutyrique est de 0,1 à 5,0.